# FASCICULE DE BREVET EUROPEEN

(11) **EP 0 900 554 B1**
(45) Date de publication et mention de la délivrance du brevet: **15.12.2004**
(21) Numéro de dépôt: 98402171.7
(22) Date de dépôt: 02.09.1998
(51) Int. Cl.: A61F 9/013

(54) **Microkératome pour chirurgie ophthalmologique**
MIKROKERATOM FÜR DIE AUGENCHIRURGIE
Microkeratome for ophthalmological surgery

(30) Priorité: 04.09.1997 FR 9711010
(43) Date de publication de la demande: 10.03.1999
(73) Titulaire: Moria SA, 92160 Antony (FR)
(72) Inventeur: Aufaure, Jean-Luc, 03210 Souvigny (FR); Duprat, Alain, 31120 Roquettes (FR)
(74) Mandataire: Robert, Jean-Pierre

(56) Documents cités:
- EP-A- 0 771 553
- WO-A-93/06783
- WO-A-98/27901
- DE-A- 3 433 581
- US-A- 5 586 980
- US-A- 5 624 456

## Description

La présente invention concerne le domaine des appareils chirurgicaux notamment destinés à l'ophtalmologie.

Parmi les techniques de chirurgie réfractive (chirurgie cornéenne) visant à la correction d'une amétropie, on connaît une technique mise au point par le Professeur Barraquer dite "Kératomileusis in situ". Cette technique consiste à retirer une partie pelliculaire de la cornée par la découpe d'un capot cornéen. Ce capot retiré, on procède ensuite au retrait d'un lenticule sur le plan découvert par le retrait du capot, lenticule dont l'épaisseur et les dimensions sont fonction de la correction à apporter. On repose ensuite le capot cornéen sur le plan ainsi modifié, ce qui se traduit par une modification de la surface rétractive externe de l'oeil.

Cette technique connaît aujourd'hui un regain d'intérêt par l'emploi de la chirurgie photoréfractive. En effet, grâce à cette chirurgie qui permet de réaliser des ablations extrêmement précises au moyen d'un laser, on a pu maîtriser les dimensions des lenticules de manière beaucoup plus fine qu'avec les moyens d'ablation mécanique en usage jusqu'alors.

Donc, dans ce type d'intervention, on retire le capot cornéen au moyen d'un microkératome, c'est-à-dire d'un outil proche d'un rabot miniaturisé avec une lame vibrante dans la direction de son fil coupant, dont on règle la profondeur de coupe au moyen de cales d'épaisseur précalibrées ou de vis micrométriques.

La chirurgie photoréfractive a permis une simplification de ces microkératomes en imposant au fil du temps une épaisseur sensiblement constante du capot cornéen. La "profondeur de coupe" étant devenue constante, le microkératome a perdu une partie de ses organes de réglage et notamment les cales calibrées ou les vis utilisées pour les réglages d'épaisseur du capot cornéen à retirer.

On a récemment proposé un microkératome dans lequel le nombre de pièces mises en oeuvre est réduit afin d'en simplifier la réalisation et surtout la préparation avant chaque intervention.

Il n'en demeure pas moins que le microkératome est encore aujourd'hui un appareil miniaturisé dont la manipulation manuelle est délicate. Le but de la présente invention est d'améliorer la sécurité de l'opération en augmentant le confort du chirurgien grâce à un appareil équipé de moyens pour améliorer la vision du champ opératoire en même temps que le maintien et la manipulation de l'appareil.

A cet effet l'invention a donc pour objet un microkératome pour chirurgie de la cornée du genre décrit dans DE 34 33 581, dans lequel l'anneau et le chariot comportent des butées de réglage de la position de fin de coulissement du chariot par rapport à l'anneau dans le sens de la coupe, le chariot comportant deux rails latéraux inférieurs pour coopérer avec la coulisse de l'anneau, l'extrémité avant du plateau étant en retrait de l'extrémité avant des rails. On a donc un microkératome dont le plateau d'aplanissement de la cornée est de largeur très courte par rapport aux microkératomes usuels.

Dans un premier mode de réalisation, la butée portée par l'anneau comporte un ergot s'élevant au-dessus du plan de la coulisse. Dans ce premier mode de réalisation la butée portée par le chariot comporte une pièce de révolution portée de manière amovible par un doigt s'élevant au-dessus du plateau d'aplanissement de la cornée et formant protubérance à l'avant de ce dernier. On peut ainsi, en changeant la dimension de cette pièce de révolution, faire varier la dimension de la portion du capot cornéen qui reste attachée à l'oeil.

Dans un autre mode de réalisation, la butée portée par l'anneau est une came à position réglable. Par exemple cette came peut être un excentrique dont la surface latérale constitue une butée pour la partie avant du chariot butée dont la position par rapport à la coulisse que porte l'anneau est réglable par rotation de cet excentrique.

Par ailleurs, pour dégager le plus possible la vision du champ opératoire par le chirurgien et pour rendre l'appareil ergonomique, la poignée de manutention de l'anneau est en forme de fourche dont les branches sont implantées inclinées dans l'anneau de part et d'autre de la coulisse. Ainsi, la poignée se trouve orientée dans la direction de la coulisse de l'anneau et à l'opposé du trajet du chariot lors de la découpe du capot cornéen. Le chirurgien manoeuvre donc le chariot par une main en direction de la poignée qu'il tient de l'autre main de manière antagoniste si bien que lorsque le chariot vient en butée contre l'arrêtoir que porte l'anneau, le chirurgien par la poignée ressent immédiatement cette butée et équilibre automatiquement l'effort que le chariot applique sur l'anneau qui tendrait à le faire basculer ce qui, dans les cas extrêmes, pourrait conduire à la rupture de la fixation par aspiration de l'anneau sur l'oeil.

Pour améliorer encore le confort du chirurgien et donc la sécurité de l'opération, le chariot du microkératome selon l'invention sera réalisé dans un matériau transparent permettant une vision totale de la découpe au travers du plateau d'aplanissement de la cornée. Enfin, de manière avantageuse, l'anneau de fixation du microkératome à l'oeil sera équipé extérieurement d'éléments de blépharostat. Habituellement ces éléments appartiennent à un mécanisme séparé dont la structure vient nécessairement encombrer la vision du champ opératoire.

D'autres caractéristiques et avantages de l'invention ressortiront des exemples de réalisation donnés dans la description ci-après à titre non limitatif.

Il sera fait référence aux dessins annexés parmi lesquels :
- la figure 1 est une vue en coupe d'un microkératome conforme à l'invention,
- la figure 2 est une vue de dessus de la figure 1,
- la figure 3 est un schéma d'un anneau de fixation appartenant au microkératome selon l'invention et équipé d'une poignée à fourche,
- la figure 4 est une vue schématique en perspective d'un anneau du microkératome selon l'invention équipé d'une poignée à fourche et d'un blépharostat.

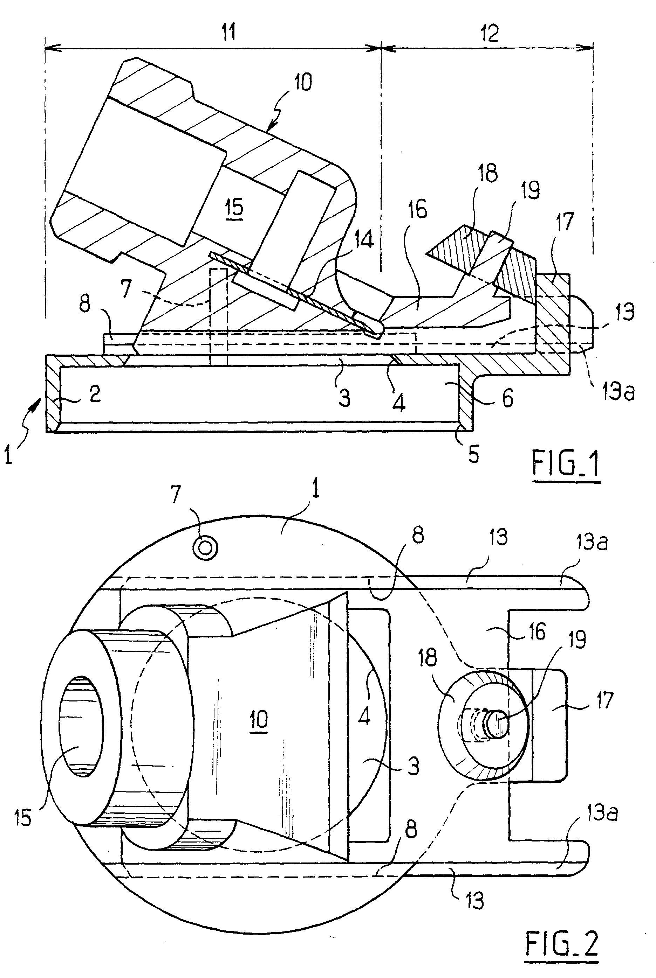

De manière classique, le microkératome représenté aux figures 1 et 2 comporte un anneau 1 pourvu d'une jupe 2 et d'une ouverture supérieure 3, la jupe et l'ouverture supérieure définissant des portées circulaires 4 et 5 qui reposent sur l'oeil de sorte qu'une chambre annulaire 6 est ainsi délimitée et peut être mise en dépression au moyen d'un embout de raccordement 7 à une source de vide non représentée. De part et d'autre de l'ouverture 3, à sa surface supérieure, l'anneau comporte deux glissières 8 qui définissent une coulisse dans laquelle un chariot 10 peut être introduit et guidé.

Le chariot 10 comporte, également classiquement, deux sections 11 et 12 bordées en partie inférieure par deux rails de guidage 13 dont le profil est conjugué au profil des glissières 8. La section 11 arrière est la section qui porte une lame de coupe 14 et son dispositif d'entraînement non représenté qui vient se loger dans un évidement 15. On rappellera brièvement que ce dispositif d'entraînement est formé par un axe de sortie d'une turbine qui possède à son extrémité libre un doigt excentré venant coopérer avec une rainure d'un talon portant la lame de coupe 14 afin de transformer le mouvement de rotation de l'axe de sortie de la turbine en un mouvement alternatif de la lame de coupe dans un plan perpendiculaire au plan de la figure.

La section avant 12 du chariot 10 comporte essentiellement un plateau 16 dont la fonction est d'aplanir à l'avant de la lame de coupe la cornée de manière à définir l'épaisseur du capot cornéen qui sera découpé par la lame 14.

Selon l'invention, l'anneau 1 est équipé d'un ergot 17 qui fait saillie au-dessus du plan de la coulisse 8 de manière à constituer une butée pour l'avant du plateau d'aplanissement 16.

En réalité, dans le mode de réalisation des figures 1 et 2, ce n'est pas à proprement parler l'avant du plateau qui vient buter contre l'ergot 17 mais un élément de butée 18 porté par le plateau 16. Dans le mode représenté, cet élément de butée 18 est ici en forme d'une rondelle conique placée sur un doigt 19 solidaire du plateau 16, l'angle du cône de la rondelle 18 et l'inclinaison vers l'avant du doigt 19 étant telle que la génératrice avant de la rondelle 18 se trouve dans le plan, ici vertical, de l'ergot 17 faisant face au chariot 10 et en protubérance par rapport à l'avant du plateau 16. En plaçant sur le doigt 19 des rondelles de diamètres différents, on peut limiter la course du chariot par rapport à l'anneau dans le sens de la découpe de la cornée à des stades différents ce qui permet d'ajuster l'importance de la découpe donc l'importance de la partie du capot cornéen restant attachée à l'oeil.

Dans une variante non représentée, le doigt 19 peut être implanté perpendiculairement au plateau 16 du chariot et la rondelle 18 peut être constituée par une rondelle strictement cylindrique. Ce n'est pas non plus sortir du cadre de l'invention que de prévoir une inversion cinématique de ces éléments de butée, c'est-à-dire de mettre en place sur l'anneau un doigt permettant d'enficher des rondelles ou des cames de diamètres différents afin de constituer des butées différentes à l'avant du plateau 16.

L'une des conséquences de la création de ces moyens de butée réside dans le fait que le plateau 16 ne s'étend pas jusqu'à l'extrémité des rails 13 du chariot 10. On constate en effet notamment à la figure 2, que ces rails débordent de l'avant du plateau 16 par leur extrémité 13a. Ces dispositions permettent de conserver une longueur de guidage suffisante notamment lorsqu'on introduit le chariot à l'intérieur de la coulisses 8, au début de l'opération de découpe de la cornée, sans pour autant imposer une extension de la dimension de l'anneau dans une direction parallèle à la coulisse 8 trop importante qui constituerait une gêne pour la mise en place de cet anneau sur l'oeil d'un patient.

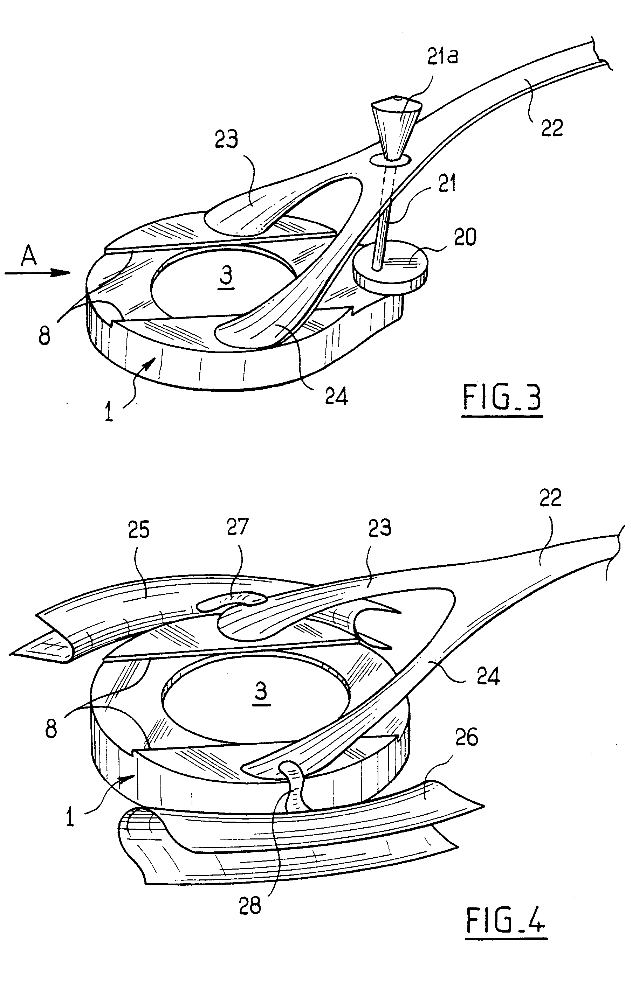

A la figure 3, on a représenté uniquement un anneau de fixation d'un kératome conforme à l'invention dans lequel l'élément de butée du chariot qui est porté par l'anneau 1 est en fait constitué par un disque 20 monté à rotation de manière excentrique sur un pivot solidaire de l'anneau 1 et susceptible d'être réglé en position angulaire autour de ce pivot au moyen d'un bouton de manoeuvre 21a d'un axe 21.

A cette figure on remarque que l'anneau est pourvu d'une poignée 22 de manutention qui est conformée en fourche et dont chacune des branches 23 et 24 est implantée de manière inclinée sur la surface supérieure de l'anneau de chaque côté de la glissière 8. Le bouton 21 de manoeuvre du disque excentrique 20 se prolonge par un axe qui se prolonge par un axe qui traverse la poignée 22, celle-ci formant un élément de guidage et de support de cet axe. On comprend de ce schéma que la section avant 12 du chariot peut librement circuler entre les branches 23, 24 de la poignée 22. Cette poignée s'étend dans une direction parallèle à celle de la coulisse 8 et permet au chirurgien une manoeuvre plus ergonomique du kératome puisque par sa main gauche il actionnera le chariot en direction de la flèche A et par sa main droite par exemple il tiendra la poignée 22 en opposition au mouvement A de sa main gauche. Cette disposition de la poignée dans l'axe de la coulisse 2 donnera également au chirurgien une information sensitive très précise lorsque le chariot parviendra en butée sur le disque excentrique 20. Enfin les branches 23 et 24 qui sont inclinées vers l'extérieur de l'anneau 1 laissent libre un champ de vision important au-dessus de cet anneau pour le chirurgien qui procède à la découpe.

A la figure 4, on a représenté schématiquement un anneau 1 appartenant au kératome selon l'invention équipé d'une poignée 22 comme décrite à la figure 3 et équipée d'éléments 25, 26 de blépharostat. Ces éléments sont représentés en forme de gouttières sensiblement curvilignes, orientées parallèlement à la coulisse 8 et attelés à l'anneau ou à la base de chacune des branches 23 et 24 de la fourche de la poignée 22 par des éléments 27 et 28 représentés de manière très schématique. En effet ces éléments 27 et 28 ont pour fonction de maintenir les gouttières 25 et 26 correctement appliquées contre les paupières afin de les écarter au maximum et sont donc des éléments qui agissent élastiquement sur les gouttières 25 et 26 pour résister à la tendance des paupières à se rapprocher l'une de l'autre. En outre, les éléments de fixation 27 et 28 peuvent disposer d'une possibilité de réglage du niveau des gouttières 25 et 26 du blépharostat par rapport à la surface de l'oeil . L'illustration de la figure 4 étant essentiellement schématique, tout moyen permettant d'atteler le blépharostat à l'anneau du kératome conforme à l'invention reste bien entendu dans le cadre de cette présente invention.

Dans le cas où le plateau 16 bute directement sur une came comme celle des figures 3 et 4, le chariot peut avantageusement être réalisé en matière transparente ce qui permet au chirurgien de surveiller précisément le comportement de la cornée lors de la découpe.

## Revendications

1. Microkératome pour chirurgie de la cornée comprenant un anneau (1) de fixation comportant sur une surface supérieure une coulisse (8) et un chariot (10) porte-lame équipé en partie inférieure de moyens (13) de guidage pour coopérer avec la coulisse (8), l'anneau comportant une poignée de manutention, le chariot porte-lame (10) comportant une section avant (12) formant plateau (16) d'aplanissement de la cornée, le chariot (10) comportant deux rails (13) latéraux inférieurs pour coopérer avec la coulisse (8) de l'anneau (1), **caractérisé en ce que** l'anneau (1) et le chariot (10) comportent des butées (17, 18) de réglage de la position de fin de coulissement du chariot (10) par rapport à l'anneau (1), et **en ce que** l'extrémité avant du plateau (16) est en retrait de l'extrémité avant (13a) des rails (13).

2. Microkératome selon la revendication 1, **caractérisé en ce que** la butée (17) portée par l'anneau comporte un ergot s'élevant au-dessus du plan de la coulisse (8), à l'avant du plateau d'aplanissement de la cornée.

3. Microkératome selon la revendication 2, **caractérisé en ce que** la butée (18) portée par le chariot comporte une pièce de révolution engagée de manière amovible sur un doigt (19) s'élevant au-dessus du plateau (16) d'aplanissement de la cornée, cette pièce formant protubérance à l'avant de ce plateau.

4. Microkératome selon la revendication 1, **caractérisé en ce que** la butée (20) portée par l'anneau (1) est une came à positions angulaires réglables.

5. Microkératome selon l'une des revendications précédentes **caractérisé en ce que** la poignée (22) de manutention de l'anneau (1) est en forme de fourche dont les branches (23) et (24) sont implantées inclinées dans l'anneau (1) de part et d'autre de la coulisse (8).

6. Microkératome selon la revendication 4 et la revendication 5, **caractérisé en ce que** la came à position réglable (20) est solidaire d'un axe de manoeuvre (21) traversant la poignée (22).

7. Microkératome selon l'une des revendications précédentes **caractérisé en ce que** le chariot (10) est en matériau transparent.

8. Microkératome selon l'une des revendications précédentes **caractérisé en ce que** l'anneau (1) est équipé de part et d'autre de la coulisse (8) d'éléments (25, 26) de blépharostat.

## Patentansprüche

1. Mikrokeratom für die Hornhautchirurgie, umfassend einen Fixierring (1), der auf einer oberen Fläche eine Führung (8) und einen messertragenden Schlitten (10) hat, der in einem unteren Teil mit Führungsmitteln (13) versehen ist, die mit der Führung (8) zusammenwirken, wobei der Ring einen Handhabungsgriff hat, der messertragende Schlitten (10) einen vorderen Abschnitt (12) hat, der eine Platte (16) zum Einebnen der Hornhaut bildet, und der Schlitten (10) zwei seitliche, untere Schienen (13) hat, die mit der Führung (8) des Rings (1) zusammenwirken, **dadurch gekennzeichnet, dass** der Ring (1) und der Schlitten (10) Anschlagvorrichtungen (17, 18) zum Einstellen der Endposition der geführten Bewegung des Schlittens (10) relativ zum dem Ring (1) umfassen, und dass das vordere Ende der Platte (16) gegenüber dem vorderen Ende (13a) der Schienen (13) zurückgesetzt ist.

2. Mikrokeratom nach Anspruch 1, **dadurch gekennzeichnet, dass** die von dem Ring getragene Anschlagvorrichtung (17) vor der zum Einebnen der Hornhaut bestimmten Platte eine Nase umfasst, die sich oberhalb der Ebene der Führung (8) erhebt.

3. Mikrokeratom nach Anspruch 2, **dadurch gekennzeichnet, dass** die von dem Schlitten getragene Anschlagvorrichtung (18) ein Rotationsstück umfasst, das abnehmbar in Eingriff mit einem Finger (19) steht, der sich oberhalb der zum Einebnen der Hornhaut bestimmten Platte (16) erhebt, wobei dieses Stück einen überstehenden Teil am Vorderteil der Platte bildet.

4. Mikrokeratom nach Anspruch 1, **dadurch gekennzeichnet, dass** die von dem Ring (1) getragene Anschlagvorrichtung (20) eine Scheibe mit einstellbaren Winkelstellungen ist.

5. Mikrokeratom nach einem der vorhergehenden Ansprüche, **dadurch gekennzeichnet, dass** der Handhabungsgriff (22) des Rings (1) als Gabel ausgebildet ist, deren Äste (23, 24) schräg in dem Ring (1) auf beiden Seiten der Führung (8) eingesetzt sind.

6. Mikrokeratom nach Anspruch 4 und Anspruch 5, **dadurch gekennzeichnet, dass** die in ihrer Stellung einstellbare Scheibe (20) einstückig mit einer Handhabungsachse (21) ausgebildet ist, die den Griff (22) durchsetzt.

7. Mikrokeratom nach einem der vorhergehenden Ansprüche, **dadurch gekennzeichnet, dass** der Schlitten (10) aus einem transparenten Material besteht.

8. Mikrokeratom nach einem der vorhergehenden Ansprüche, **dadurch gekennzeichnet, dass** der Ring (1) auf beiden Seiten der Führung (8) mit Blepharostatelementen (25, 26) versehen ist.

## Claims

1. Microkeratome for surgery of the cornea, comprising a securing ring (1) which includes, on an upper surface, a slide (8), and a blade carriage (10) equipped in the lower part with guide means (13) for cooperating with the slide (8), the ring including a manoeuvring grip, the blade carriage (10) including a front section (12) forming a plateau (16) for flattening the cornea, the carriage (10) including two lower side rails (13) for cooperating with the slide (8) of the ring (1), **characterized in that** the ring (1) and the carriage (10) include abutments (17, 18) for regulating the end of slide position of the carriage (10) in relation to the ring (1) and **in that** the front end of the plateau (16) is set back from the front end (13a) of the rails (13).

2. Microkeratome according to claim 1, **characterized in that** the abutment (17) carried by the ring includes a lug rising above the plane of the slide (8).

3. Microkeratome according to claim 2, **characterized in that** the abutment (18) carried by the carriage includes a component of revolution engaged movably on a finger (19) rising above the plateau (16) for flattening the cornea, this component forming a protrusion to the front of this plateau.

4. Microkeratome according to claim 1, **characterized in that** the abutment (20) carried by the ring (1) is a cam with adjustable positions.

5. Microkeratome according to one of the preceding claims, **characterized in that** the manoeuvring grip (22) of the ring (1) is in the shape of a fork whose prongs (23) and (24) are implanted at an inclination in the ring (1) on either side of the slide (8).

6. Microkeratome according to claim 4 and claim 5, **characterized in that** the cam (20) with adjustable positions is integral with a manoeuvring pin (21) passing through the grip (22).

7. Microkeratome according to one of the preceding claims, **characterized in that** the carriage (10) is made of transparent material.

8. Microkeratome according to one of the preceding claims, **characterized in that** the ring (1) is equipped on either side of the slide (8) with eye speculum elements (25, 26).
